# FASCICULE DE BREVET EUROPEEN

(11) **EP 2 593 135 B1**
(45) Date de publication et mention de la délivrance du brevet: **03.01.2018**
(21) Numéro de dépôt: 11731336.1
(22) Date de dépôt: 08.07.2011
(51) Int. Cl.: A61K 39/145, C07K 14/11, C12N 7/04, C12N 7/01, C12N 9/12

(54) **SOUCHES VIRALES MODIFIÉES ET PROCÉDÉ POUR AMÉLIORER LA PRODUCTION DE SEMENCES VACCINALES DE VIRUS INFLUENZA**
MODIFIZIERTE VIRENSTÄMME UND VERFAHREN ZUR VERBESSERUNG DER HERSTELLUNG VON IMPFKRISTALLEN DES INFLUENZA-VIRUS
MODIFIED VIRAL STRAINS, AND METHOD FOR IMPROVING THE PRODUCTION OF VACCINE SEEDS OF THE INFLUENZA VIRUS

(30) Priorité: 13.07.2010 FR 1055716
(43) Date de publication de la demande: 22.05.2013
(73) Titulaire: Université Claude Bernard Lyon 1, 69100 Villeurbanne (FR); Hospices Civils de Lyon, 69002 Lyon (FR); Centre National de la Recherche Scientifique (CNRS), 75016 Paris (FR)
(72) Inventeur: MOULES, Vincent, 69220 Belleville (FR); ROSA-CALATRAVA, Manuel, 69003 Lyon (FR); FERRARIS, Olivier, 69500 Bron (FR); YVER, Matthieu, 75016 Paris (FR)
(74) Mandataire: Regimbeau
(86) Numéro de dépôt international: PCT/EP2011/061604
(87) Numéro de publication internationale: WO 2012/007380

(56) Documents cités:
- US-A1- 2009 074 804
- WANITCHANG A ET AL: "Enhancement of reverse genetics-derived swine-origin H1N1 influenza virus seed vaccine growth by inclusion of indigenous polymerase PB1 protein", VIRUS RESEARCH, AMSTERDAM, NL, vol. 147, no. 1, 1 janvier 2010 (2010-01-01), pages 145-148, XP026793625, ISSN: 0168-1702 [extrait le 2009-10-30] cité dans la demande -& DATABASE EMBL [Online] E.B.I. Hinxton U.K.; 15 mai 2009 (2009-05-15), Chitakanpitch M et al: "polymerase PB1 [Influenza A virus (A/Nonthaburi/102/2009(H1N1))].", XP002629931, Database accession no. ACR08608
- DATABASE EMBL [Online] E.B.I. Hinxton U.K.; 28 mars 2008 (2008-03-28), Bragstad K et al: "Influenza A virus (A/Moscow/10/1999(H3N2)) polymerase PB1 (PB1) gene, complete cds", XP002629932, Database accession no. EU097800
- LEE KWANG-HEE ET AL: "The position 4 nucleotide at the 3' end of the influenza virus neuraminidase vRNA is involved in the temporal regulation of transcription and replication of neuraminidase RNAs and affects the repertoire of influenza virus surface antigens", JOURNAL OF GENERAL VIROLOGY, vol. 79, no. 8, août 1998 (1998-08), pages 1923-1934, XP002629933, ISSN: 0022-1317
- MOULES V ET AL: "INFLUENZA VIRION MORPHOLOGIES EXPLAINED BYCELLULAR ORIGIN, SUB-TYPES AND GENETICBACKGROUND:IMPLICATIONS FOR VACCINE DESIGN ANDPATHOGENESIS STUDIES", 7 avril 2010 (2010-04-07), 4TH EUROPEAN CONGRESS OF VIROLOGY - ABSTRACTS, EUROPEAN SOCIETY FOR VIROLOGY, PAGE(S) 86, XP002625913, le document en entier
- VINCENT MOULÈS ET AL: "Importance of viral genomic composition in modulating glycoprotein content on the surface of influenza virus particles", VIROLOGY, ELSEVIER, AMSTERDAM, NL, vol. 414, no. 1, 14 March 2011 (2011-03-14), pages 51-62, XP028206868, ISSN: 0042-6822, DOI: 10.1016/J.VIROL.2011.03.011 [retrieved on 2011-03-16]

### SEQUENCE LISTING

<110> Université Claude Bernard Lyon 1 Hospices Civils de Lyon Centre National de la Recherche Scientifique (CNRS)
<120> Souches Virales modifiées et procédé pour améliorer la production de semences vaccinales de virus influenza
<130> D28324 KH
<130> FR 1055716
<130> 2010-07-13
<160> 2
<170> PatentIn version 3.3
<210> 1
   <211> 757
   <212> PRT
   <213> Influenza A virus
<400> 1
   Met Asp Val Asn Pro Thr Leu Leu Phe Leu Lys Val Pro Ala Gln Asn
   1               5                   10                  15
   Ala Ile Ser Thr Thr Phe Pro Tyr Thr Gly Asp Pro Pro Tyr Ser His
               20                  25                  30
   Gly Thr Gly Thr Gly Tyr Thr Met Asp Thr Val Asn Arg Thr His Gln
           35                  40                  45
   Tyr Ser Glu Lys Gly Arg Trp Thr Thr Asn Thr Glu Thr Gly Ala Pro
       50                  55                  60
   Gln Leu Asn Pro Ile Asp Gly Pro Leu Pro Glu Asp Asn Glu Pro Ser
   65                  70                  75                  80
   Gly Tyr Ala Gln Thr Asp Cys Val Leu Glu Ala Met Ala Phe Leu Glu
                   85                  90                  95
   Glu Ser His Pro Gly Ile Phe Glu Asn Ser Cys Ile Glu Thr Met Glu
               100                 105                 110
   Val Val Gln Gln Thr Arg Val Asp Lys Leu Thr Gln Gly Arg Gln Thr
           115                 120                 125
   Tyr Asp Trp Thr Leu Asn Arg Asn Gln Pro Ala Ala Thr Ala Leu Ala
       130                 135                 140
   Asn Thr Ile Glu Val Phe Arg Ser Asn Gly Leu Thr Ala Asn Glu Ser
   145                 150                 155                 160
   Gly Arg Leu Ile Asp Phe Leu Lys Asp Val Met Glu Ser Met Asn Lys
                   165                 170                 175
   Glu Glu Met Gly Ile Thr Thr His Phe Gln Arg Lys Arg Arg Val Arg
               180                 185                 190
   Asp Asn Met Thr Lys Lys Met Ile Thr Gln Arg Thr Met Gly Lys Lys
           195                 200                 205
   Lys Gln Arg Leu Asn Lys Arg Ser Tyr Leu Ile Arg Ala Leu Thr Leu
       210                 215                 220
   Asn Thr Met Thr Lys Asp Ala Glu Arg Gly Lys Leu Lys Arg Arg Ala
   225                 230                 235                 240
   Ile Ala Thr Pro Gly Met Gln Ile Arg Gly Phe Val Tyr Phe Val Glu
                   245                 250                 255
   Thr Leu Ala Arg Ser Ile Cys Glu Lys Leu Glu Gln Ser Gly Leu Pro
               260                 265                 270
   Val Gly Gly Asn Glu Lys Lys Ala Lys Leu Ala Asn Val Val Arg Lys
           275                 280                 285
   Met Met Thr Asn Ser Gln Asp Thr Glu Leu Ser Phe Thr Ile Thr Gly
       290                 295                 300
   Asp Asn Thr Lys Trp Asn Glu Asn Gln Asn Pro Arg Met Phe Leu Ala
   305                 310                 315                 320
   Met Ile Thr Tyr Met Thr Arg Asn Gln Pro Glu Trp Phe Arg Asn Val
                   325                 330                 335
   Leu Ser Ile Ala Pro Ile Met Phe Ser Asn Lys Met Ala Arg Leu Gly
               340                 345                 350
   Lys Gly Tyr Met Phe Glu Ser Lys Ser Met Lys Leu Arg Thr Gln Ile
           355                 360                 365
   Pro Ala Glu Met Leu Ala Ser Ile Asp Leu Lys Tyr Phe Asn Asp Ser
       370                 375                 380
   Thr Arg Lys Lys Ile Glu Lys Ile Arg Pro Leu Leu Ile Glu Gly Thr
   385                 390                 395                 400
   Ala Ser Leu Ser Pro Gly Met Met Met Gly Met Phe Asn Met Leu Ser
                   405                 410                 415
   Thr Val Leu Gly Val Ser Ile Leu Asn Leu Gly Gln Lys Arg Tyr Thr
               420                 425                 430
   Lys Thr Thr Tyr Trp Trp Asp Gly Leu Gln Ser Ser Asp Asp Phe Ala
           435                 440                 445
   Leu Ile Val Asn Ala Pro Asn His Glu Gly Ile Gln Ala Gly Val Asp
       450                 455                 460
   Arg Phe Tyr Arg Thr Cys Lys Leu Leu Gly Ile Asn Met Ser Lys Lys
   465                 470                 475                 480
   Lys Ser Tyr Ile Asn Arg Thr Gly Thr Phe Glu Phe Thr Ser Phe Phe
                   485                 490                 495
   Tyr Arg Tyr Gly Phe Val Ala Asn Phe Ser Met Glu Leu Pro Ser Phe
               500                 505                 510
   Gly Val Ser Gly Ile Asn Glu Ser Ala Asp Met Ser Ile Gly Val Thr
           515                 520                 525
   Val Ile Lys Asn Asn Met Ile Asn Asn Asp Leu Gly Pro Ala Thr Ala
       530                 535                 540
   Gln Met Ala Leu Gln Leu Phe Ile Lys Asp Tyr Arg Tyr Thr Tyr Arg
   545                 550                 555                 560
   Cys His Ile Gly Asp Thr Gln Ile Gln Thr Arg Arg Ser Phe Glu Ile
                   565                 570                 575
   Lys Lys Leu Trp Glu Gln Thr Arg Ser Lys Ala Gly Leu Leu Val Ser
               580                 585                 590
   Asp Gly Gly Pro Asn Leu Tyr Asn Ile Arg Asn Leu His Ile Pro Glu
           595                 600                 605
   Val Cys Leu Lys Trp Glu Leu Met Asp Glu Asp Tyr Gln Gly Arg Leu
       610                 615                 620
   Cys Asn Pro Leu Asn Pro Phe Val Ser His Lys Glu Ile Glu Ser Met
   625                 630                 635                 640
   Asn Asn Ala Val Met Met Pro Ala His Gly Pro Ala Lys Asn Met Glu
                   645                 650                 655
   Tyr Asp Ala Val Ala Thr Thr His Ser Trp Ile Pro Lys Arg Asn Arg
               660                 665                 670
   Ser Ile Leu Asn Thr Ser Gln Arg Gly Val Leu Glu Asp Glu Gln Met
           675                 680                 685
   Tyr Gln Arg Cys Cys Asn Leu Phe Glu Lys Phe Phe Pro Ser Ser Ser
       690                 695                 700
   Tyr Arg Arg Pro Val Gly Ile Ser Ser Met Val Glu Ala Met Val Ser
   705                 710                 715                 720
   Arg Ala Arg Ile Asp Ala Arg Ile Asp Phe Glu Ser Gly Arg Ile Lys
                   725                 730                 735
   Lys Glu Glu Phe Thr Glu Ile Met Lys Ile Cys Ser Thr Ile Glu Glu
               740                 745                 750
   Leu Arg Arg Gln Lys
           755
<210> 2
   <211> 757
   <212> PRT
   <213> Influenza A virus
<400> 2
   Met Asp Val Asn Pro Thr Leu Leu Phe Leu Lys Val Pro Ala Gln Asn
   1               5                   10                  15
   Ala Ile Ser Thr Thr Phe Pro Tyr Thr Gly Asp Pro Pro Tyr Ser His
               20                  25                  30
   Gly Thr Gly Thr Gly Tyr Thr Met Asp Thr Val Asn Arg Thr His Gln
           35                  40                  45
   Tyr Ser Glu Lys Gly Lys Trp Thr Thr Asn Thr Glu Thr Gly Ala Pro
       50                  55                  60
   Gln Leu Asn Pro Ile Asp Gly Pro Leu Pro Glu Asp Asn Glu Pro Ser
   65                  70                  75                  80
   Gly Tyr Ala Gln Thr Asp Cys Val Leu Glu Ala Met Ala Phe Leu Glu
                   85                  90                  95
   Glu Ser His Pro Gly Ile Phe Glu Asn Ser Cys Leu Glu Thr Met Glu
               100                 105                 110
   Val Val Gln Gln Thr Arg Val Asp Lys Leu Thr Gln Gly Arg Gln Thr
           115                 120                 125
   Tyr Asp Trp Thr Leu Asn Arg Asn Gln Pro Ala Ala Thr Ala Leu Ala
       130                 135                 140
   Asn Thr Ile Glu Val Phe Arg Ser Asn Gly Leu Thr Ala Asn Glu Ser
   145                 150                 155                 160
   Gly Arg Leu Ile Asp Phe Leu Lys Asp Val Met Glu Ser Met Asp Lys
                   165                 170                 175
   Glu Glu Met Glu Ile Thr Thr His Phe Gln Arg Glu Arg Arg Val Arg
               180                 185                 190
   Asp Asn Met Thr Lys Lys Met Val Thr Gln Arg Thr Ile Gly Lys Lys
           195                 200                 205
   Lys Gln Arg Val Asn Lys Arg Gly Tyr Leu Ile Arg Ala Leu Thr Leu
       210                 215                 220
   Asn Thr Met Thr Lys Asp Ala Glu Arg Gly Lys Leu Lys Arg Arg Ala
   225                 230                 235                 240
   Ile Ala Thr Pro Gly Met Gln Ile Arg Gly Phe Val Tyr Phe Val Glu
                   245                 250                 255
   Thr Leu Ala Arg Ser Ile Cys Glu Lys Leu Glu Gln Ser Gly Leu Pro
               260                 265                 270
   Val Gly Gly Asn Glu Lys Lys Ala Lys Leu Ala Asn Val Val Arg Lys
           275                 280                 285
   Met Met Thr Asn Ser Gln Asp Thr Glu Leu Ser Phe Thr Ile Thr Gly
       290                 295                 300
   Asp Asn Thr Lys Trp Asn Glu Asn Gln Asn Pro Arg Met Phe Leu Ala
   305                 310                 315                 320
   Met Ile Thr Tyr Ile Thr Lys Asn Gln Pro Glu Trp Phe Arg Asn Ile
                   325                 330                 335
   Leu Ser Ile Ala Pro Ile Met Phe Ser Asn Lys Met Ala Arg Leu Gly
               340                 345                 350
   Lys Gly Tyr Met Phe Glu Ser Lys Arg Met Lys Leu Arg Thr Gln Ile
           355                 360                 365
   Pro Ala Glu Met Leu Ala Ser Ile Asp Leu Lys Tyr Phe Asn Glu Ser
       370                 375                 380
   Thr Arg Lys Lys Ile Glu Lys Ile Arg Pro Leu Leu Ile Asp Gly Thr
   385                 390                 395                 400
   Ala Ser Leu Ser Pro Gly Met Met Met Gly Met Phe Asn Met Leu Ser
                   405                 410                 415
   Thr Val Leu Gly Val Ser Ile Leu Asn Leu Gly Gln Lys Lys Tyr Thr
               420                 425                 430
   Lys Thr Thr Tyr Trp Trp Asp Gly Leu Gln Ser Ser Asp Asp Phe Ala
           435                 440                 445
   Leu Ile Val Asn Ala Pro Asn His Glu Gly Ile Gln Ala Gly Val Asp
       450                 455                 460
   Arg Phe Tyr Arg Thr Cys Lys Leu Val Gly Ile Asn Met Ser Lys Lys
   465                 470                 475                 480
   Lys Ser Tyr Ile Asn Lys Thr Gly Thr Phe Glu Phe Thr Ser Phe Phe
                   485                 490                 495
   Tyr Arg Tyr Gly Phe Val Ala Asn Phe Ser Met Glu Leu Pro Ser Phe
               500                 505                 510
   Gly Val Ser Gly Ile Asn Glu Ser Ala Asp Met Ser Ile Gly Val Thr
           515                 520                 525
   Val Ile Lys Asn Asn Met Ile Asn Asn Asp Leu Gly Pro Ala Thr Ala
       530                 535                 540
   Gln Met Ala Leu Gln Leu Phe Ile Lys Asp Tyr Arg Tyr Thr Tyr Arg
   545                 550                 555                 560
   Cys His Arg Gly Asp Thr Gln Ile Gln Thr Arg Arg Ser Phe Glu Leu
                   565                 570                 575
   Lys Lys Leu Trp Asp Gln Thr Gln Ser Arg Ala Gly Leu Leu Val Ser
               580                 585                 590
   Asp Gly Gly Pro Asn Leu Tyr Asn Ile Arg Asn Leu His Ile Pro Glu
           595                 600                 605
   Val Cys Leu Lys Trp Glu Leu Met Asn Glu Asp Tyr Arg Gly Arg Leu
       610                 615                 620
   Cys Asn Pro Leu Asn Pro Phe Val Ser His Lys Glu Ile Glu Ser Val
   625                 630                 635                 640
   Asn Asn Ala Val Val Met Pro Ala His Gly Pro Ala Lys Ser Met Glu
                   645                 650                 655
   Tyr Asp Ala Val Ala Thr Thr His Ser Trp Ile Pro Lys Arg Asn Arg
               660                 665                 670
   Ser Ile Leu Asn Thr Ser Gln Arg Gly Ile Leu Glu Asp Glu Gln Met
           675                 680                 685
   Tyr Gln Lys Cys Cys Asn Leu Phe Glu Lys Phe Phe Pro Ser Ser Ser
       690                 695                 700
   Tyr Arg Arg Pro Val Gly Ile Ser Ser Met Val Glu Ala Met Val Ser
   705                 710                 715                 720
   Arg Ala Arg Ile Asp Ala Arg Ile Asp Phe Glu Ser Gly Arg Ile Lys
                   725                 730                 735
   Lys Glu Glu Phe Ser Glu Ile Met Lys Ile Cys Ser Thr Ile Glu Glu
               740                 745                 750
   Leu Arg Arg Gln Lys
           755

## Description

La présente invention se rapporte à des virus influenza A/PR/8/34 modifiés portant un gène PB1 modifié ainsi qu'à des procédés mettant en oeuvre des virus réassortants ayant le fond génétique des virus A/PR/8/34 modifiés pour la production de semences vaccinales de virus influenza.

La grippe est une infection respiratoire virale fréquente, observée partout dans le monde, évoluant par bouffées épidémiques hivernales dans les régions tempérées, due aux virus influenza. Elle reste de nos jours la deuxième cause de mortalité infectieuse après les pneumonies. Les virus influenza responsables de pathologie chez l'homme sont les virus influenza de typa A et B. Alors que les virus influenza de type B circulent sous forme de lignage, les virus influenza de type A sont classés en sous types viraux en fonction des propriétés antigéniques des deux glycoprotéines majeures de surface, l'hémagglutinine (HA) et la neuraminidase (NA). Les virus influenza présentent entre 300 et 700 glycoprotéines à leur surface associée à un ratio théorique NA/HA de un pour dix. Les virus circulants chez l'homme et responsables des épidémies saisonnières sont les virus A (H1N1) et A (H3N2). Le principal réservoir des virus influenza étant le réservoir animal (aviaire et porcin), des virus animaux peuvent franchir la barrière d'espèce et infecter l'homme. Des virus comme le virus aviaire hautement pathogène A (H5N1) et le virus A (H1N1) pandémique peuvent entrainer de graves problèmes de santé publique.

La vaccination est, pour l'instant, le seul moyen efficace pour protéger les populations des virus influenza. Le vaccin dit saisonnier permet d'acquérir une immunité contre les virus circulants saisonniers A (H1N1), A (H3N2) et les virus B. Il est défini chaque année par l'OMS à partir des souches prototypes de l'année antérieure. La réponse immunitaire de l'hôte est principalement de type humorale avec synthèse d'anticorps neutralisants qui sont dirigés contre les protéines HA et NA. En raison d'une dérive antigénique importante de ces deux protéines, en particuliers pour les virus de type A, la composition vaccinale doit être réévaluée annuellement.

Le processus classique de production de vaccin repose dans un premier temps sur l'obtention par réassortiment génétique avec la souche A/PR8/34 (H1N1), de semences vaccinales sur oeuf, pour chacune des 3 souches prototypes déterminées par l'OMS. La plupart des fabricants de vaccin utilisent de manière usuelle ces virus réassortants dérivant principalement du virus parental A/PR/8/34. Ainsi, chaque semence vaccinale est issue d'un processus de réassortiment génétique entre la souche prototype et le virus A/PR8/34 (H1N1) qui possède des capacités réplicatives optimales en oeuf.

La particule virale contient huit gènes distincts constitués d'une chaîne unique d'ARN, chacun des gènes codant une à trois protéines déterminées du virus : HA, NA, M1, M2, NP, NS1, NS2, PB1, PB1F2, PB1N40, PB2 et PA. Par conséquent, un long processus de sélection permet l'obtention du réassortant vaccinal, comportant au minimum les segments de gènes codant pour HA et NA de la souche prototype sur le fond génétique du virus PR8 (composition « 6+2 »). Les 3 réassortant vaccinaux, issus du réassortiment génétique entre les 3 souches prototypes et la souche parentale A/PR/8/34 sont ensuite amplifiées sur oeuf. Les antigènes HA et NA sont purifiés à partir des productions allantoïques, associés ou non avec des adjuvants afin de produire les doses vaccinales. Ce processus industriel pour la fabrication des doses vaccinales est long (4 à 6 mois). Des stratégies alternatives pour l'obtention des doses vaccinales se sont développées ces dernières années. En effet, l'utilisation de lignée cellulaire pour l'amplification des reassortants vaccinaux permet entre autre de ne plus être dépendant du système « oeuf » (quantité d'oeuf pas suffisante pour la gestion d'une pandémie), réduit les modifications des antigènes de surface régulièrement observées dans la production allantoïque et n'entraine pas d'allergies. Cependant, à l'heure actuelle, peu d'industriels ont choisis ce nouveau mode de production car le processus industriel est loin d'etre aussi performant que les oeufs pour l'instant.

Afin de réduire au minimum le temps de production des doses vaccinales et avec l'objectif d'avoir un niveau de rendement équivalent, en terme de nombre de dose, l'obtention des semences vaccinales peut être réalisée par l'utilisation des techniques de génétique inverse, permettant d'obtenir rapidement le réassortant vaccinal de composition "6+2", éliminant ainsi toutes les étapes de sélection. La production de virus recombinés par génétique inverse présente l'alternative la plus réaliste pour répondre efficacement à la demande de vaccins. La production de virus recombinés par génétique inverse donne la possibilité, dans un deuxième temps, de produire un virus PR8 « optimisé » permettant, lorsqu'il sera utilisé par un processus de réassortiment génétique avec des souches prototypes, de produire des reassortants vaccinaux présentant des caractéristiques virales optimisées pour la production de dose vaccinale en oeuf ou en cellule.

Dans le contexte actuel de l' émergence d'un virus pathogène pandémique l'infection grippale pourrait entrainer 1,3 à 2 millions d'hospitalisations et de 280 000 à 650 000 décès dans les seuls pays industrialisés (données OMS).

La dose vaccinale est définie par une quantité d'antigène HA fixe (15 microgramme /sous-type viral/dose). Une détection positive de l'antigène NA est nécessaire pour la validation des lots vaccinaux. Un des enjeux économiques majeurs est de pouvoir réduire le coût de fabrication d'une dose vaccinale (plus de doses par production et/ou réduction du temps d'obtention de la même quantité de doses).

Wanitchang *et al.* décrivent l'amélioration de la croissance et l'augmentation de l'activité NA de virus réassortant exprimant les glycoprotéines HA/NA du virus H1N1 (S-OIV) lorsque le gène PB1 du virus A/PR/8/34 réassortant (« 5+3 ») est remplacé par le gène PB1 du virus S-OIV. L'amélioration de la croisssance et l'augmentation de l'activité NA sont cependant spécifiques des glycoprotéines HA/NA de S-OIV. En outre, l'amélioration de croissance et l'augmentation de l'activité NA ne sont pas détectées chez un virus A/PR/8/34 réassortant (« 7+1 ») portant le gène PB1 de S-OIV. Ce document ne décrit pas de procédés pour accroître la quantité de glycoprotéines HA-NA à la surface d'un virus A/PR/8/34 et il ne décrit pas non plus de virus A/PR/8/34 modifiés dont la quantité de glycoprotéines HA-NA en surface du virus est accrue.

La présente invention concerne des souches virales (réassortants vaccinaux) produisant plus de glycoprotéines de surface et des procédés pour améliorer la production des glycoprotéines de surface HA/NA. L'invention consiste notamment en la modification de virus PR8 usuellement utilisés pour la production des semences vaccinales du virus de la grippe. Cette modification consiste en la modification de certains acides aminés de la protéine PB 1 afin d'obtenir des réassortants vaccinaux présentant une plus grande quantité de glycoprotéines de surface (HA et NA) et un ratio HA/NA différent en faveur de la NA. Celles-ci constituent les antigènes des doses vaccinales anti-influenza. L'invention permet d'optimiser quantitativement et qualitativement les semences vaccinales des virus influenza de type A et B.

Un des avantages de l'invention est d'augmenter l'immunogénicité des doses vaccinales par surexpression de NA par rapport à une quantité donnée de HA produite.

Un autre avantage est de disposer d'un système qui permettra d'augmenter le nombre de doses vaccinales produites sur oeuf ou cellule par l'augmentation de l'expression des glycoprotéines de surface.

### RESUME DE L'INVENTION

L'invention a pour objet des virus influenza A/PR/8/34 modifiés, dont la quantité de glycoprotéines HA-NA (hémagglutinine et neuraminidase) à la surface est supérieure à 550 glycoprotéines pour un virion de 100 nm de diamètre, comprenant un gène PB1 codant pour une protéine PB1 ayant la séquence SeqIdNo.2, et les gènes HA et NA dudit virus influenza A/PR/8/34 ou les gènes HA et NA d'un virus influenza ayant les sous-types H3N2, H5N1 ou H5N2. Un autre objet de la présente invention est un procédé pour produire des glycoprotéines HA-NA vaccinales de virus influenza dans lequel on amplifie sur oeuf ou cellule ex vivo un virus influenza modifié selon l'invention et comprenant les gènes HA et NA codant pour lesdites glycoprotéines HA-NA vaccinales. Les glycoprotéines HA-NA vaccinales sont choisies parmi les glycoprotéines des virus ayant les sous-types H3N2, H5N2, H5N1. L'invention se rapporte également à un procédé pour accroître la quantité de glycoprotéines HA-NA à la surface d'un virus influenza A/PR/8/34 caractérisé en ce qu'il comprend le remplacement du gène PB1 codant pour une protéine PB1 de séquence SeqIdNo.1 dudit virus influenza A/PR/8/34 par le gène PB1 d'un virus influenza H3N2 codant pour une protéine PB1 ayant la séquence de la SEQ ID No. 2, et en ce que le-dit virus influenza A/PR/8/34 comprend les gènes HA et NA dudit virus influenza A/PR/8/34 ou les gènes HA et NA d'un virus influenza ayant les sous-types H3N2, H5N1 ou H5N2. L'invention a également pour objet un procédé pour accroître l'immunogénicité d'une dose vaccinale comprenant des glycoprotéines HA-NA de virus influenza en élevant le rapport NA/HA des glycoprotéines produites dans lequel les glycoprotéines sont produites par amplification sur oeufs ou cellules ex vivo d'un virus influenza modifié selon l'invention.

### LISTAGE DE SEQUENCES

SEQ ID No.1 : Séquence protéique de PB1 de virus influenza A/PR8/8/34 A(H1N1)
SEQ ID No.2 : Séquence protéique de PB1 de virus influenza Moscow/10/99 A(H3N2)

### DESCRIPTION DE L'INVENTION

L'invention repose sur l'observation inattendue que différentes souches de virus influenza n'ont pas la même quantité de glycoprotéines HA et NA à leur surface. En particulier, il s'est avéré que les virus influenza ayant le fond génétique de la souche mère A/PR/8/34, utilisé par les industriels pour l'amplification virale par injection dans des oeufs fertilisés de poulet, expriment une plus faible quantité de glycoprotéines HA et NA à leur surface que d'autres souches de virus influenza. Cette constatation a une grande importance car des virus réassortants ayant un fond génétique A/PR/8/34 et exprimant des glycoprotéines HA et NA vaccinales sont classiquement utilisés pour la production de vaccins antigrippaux. Une dose vaccinale étant définie par une quantité d'antigène HA fixe de 15 µg/sous-type viral/dose, l'augmentation de la quantité de glycoprotéines produites par les virus influenza ayant un fond génétique A/PR/8/34 est un enjeu majeur pour améliorer le rendement de la production de doses vaccinales et diminuer le coût de fabrication de ces doses vaccinales.

Au vu de ces résultats, il a maintenant été trouvé de façon inattendue que la quantité de glycoprotéines HA-NA produites par un virus ayant un fond génétique A/PR/8/34 peut être accrue par la modification génétique du gène PB1 de ce virus. La présente invention se rapporte ainsi à des virus modifiés ayant un fond génétique A/PR/8/34 produisant une quantité plus importante de glycoprotéines HA-NA de souches prototypes ainsi qu'à des procédés pour accroître le rendement de la production de glycoprotéines vaccinales HA-NA et à des procédés pour accroître la quantité de glycoprotéines produites par un virus A/PR/8/34. La présente invention se rapporte aussi à des virus modifiés ayant un fond génétique A/PR/8/34 ayant un ratio HA/NA différent en faveur de NA.

L'invention se rapporte en particulier à des virus A/PR/8/34 modifiés comprenant un gène PB1 modifié ainsi qu'à des virus A/PR/8/34 modifiés réassortants comprenant un gène PB1 modifié et les gènes HA et NA d'un autre virus et notamment d'un virus prototype pour la production d'antigènes HA/NA vaccinaux. L'invention concerne également des procédés pour produire des glycoprotéines HA-NA comprenant l'amplification sur oeufs des virus modifiés ou des virus réassortants selon l'invention. Enfin, l'invention a aussi pour objet des procédés pour accroître significativement la quantité de glycoprotéines HA-NA à la surface d'un virus A/PR/8/34 ou d'un virus réassortant ayant un fond génétique A/PR/8/34. Ces procédés permettent d'améliorer le rendement de la production de glycoprotéines HA-NA.

Par « virus influenza A/PR/8/34 », on entend des virus apparentés à la souche A/PR/8/34. La souche A/PR/8/34 résulte de l'adaptation à la culture sur oeufs de virus influenza, prélevés sur un patient à Puerto Rico en 1934. Différents clones de la souche A/PR/8/34 ont été développés et sont bien connus de l'homme du métier. La souche A/PR/8/34, ainsi que différents clones dérivés de cette souche, ont fait l'objet de dépôts à l'ATCC. Il s'agit notamment des dépôts ATCC VR-95 et VR-1469. Cette souche A/PR/8/34 est utilisée comme souche mère pour la préparation de virus réassortants ayant un haut potentiel de multiplication sur oeufs pour la production vaccinale. Différents clones de la souche A/PR/8/34 sont utilisés par les industriels mais ces clones demeurent génétiquement très proches et sont caractérisés par une forte capacité de croissance sur oeufs pour la production de vaccin. Les sequences des 8 segments de genes du virus A/PR/8/34 sont disponibles dans GENBANK sous les numéros suivants (HA : FJ888348.1, NA: CY038905.1, PB2 : CY040177.1, PA : CY038908.1, NS : CY040174.1, PB1 : CY040176.1, NP : CY040173.1 et M : CY040171.1).

Par « virus influenza A/PR/8/34 modifié », on entend un virus apparenté à la souche A/PR/8/34 dont le gène PB1 est modifié.

Les virus influenza de type A comprennent huit gènes dont notamment les gènes PB1, HA et NA.

Par « virus réassortant ayant le fond génétique du virus A/PR/8/34 modifié », on entend un virus réassortant possédant 6 gènes du virus A/PR/8/34 modifié, y compris le gène PB1 modifié, et les gènes codant pour les glycoprotéines HA et NA d'une autre souche de virus influenza. Les gènes codant pour les glycoprotéines HA et NA proviennent en général de virus grippaux de type sauvage (ou virus prototype) qui ont été sélectionnés comme étant représentatifs de groupes importants de virus grippaux sur la base d'études antigéniques et génétiques approfondies (par les laboratoires associés à L'OMS). Ces virus grippaux de type sauvage (virus prototype) sont cultivés directement à partir d'échantillons cliniques. Chaque année l'OMS recommande ainsi de préparer un vaccin antigrippal à partir de certains virus grippaux de type sauvage. Les virus réassortants correspondants ayant le fond génétique du virus A/PR/8/34 modifié ont une forte capacité de croissance sur oeufs (caractéristique de la souche A/PR/8/34) et expriment des glycoprotéines HA et NA pour la production vaccinale.

Les termes « virus réassorants » et « réassortiment génétique» font référence à un processus par lequel les gènes de deux ou plusieurs virus grippaux se retrouvent dans des combinaisons différentes, pour donner des virus hybrides possédant des gènes de chacun des virus parentaux. Ce réassortiment génétique peut se produire naturellement ou être obtenu en laboratoire.

Les virus influenza A/PR/8/34 modifiés selon l'invention comprennent un gène PB1 (protéine basique 1) modifié codant pour une protéine PB1 ayant la séquence SeqIdNo.2. Le gène PB1 des virus influenza de type A est bien connu de l'homme du métier. La séquence du gène PB1 de A/PR/8/34 est par exemple disponible dans GENBANK sous les numéros (CY040176.1, CY038909.1, CY038901.1, EF190980.1, EF190972.1).

Les gènes PB1 de différents clones de virus influenza A/PR/8/34 pourront présenter un petit nombre de divergences de séquence même si la dérive génétique pour le gène PB1 est faible.

Par « gène PB1 modifié », on entend un gène PB1 portant des modifications au niveau de sa séquence entrainant une différence dans les séquence d'acides aminés des protéines codées par ce segment.

Le gene PB1 code pour 3 protéines (PB1, PB1F2 et PB1N40), la protéine PB1 est bien connue et joue un rôle central dans la réplication/transcription du virus (sans cette protéine, pas de réplication). Les autres protéines sont dites auxiliaires et sont mal connues. La protéine PB1F2 semble joue un rôle in vivo tandis qu'une seule étude décrit un role putatif de la protéine PB1N40 dans la transcription. De plus, tous les segments de gènes PB1 des virus circulants ne codent pas tous pour la protéine PB1F2. L'homme du métier comprendra que des modifications du gène PB1 peuvent donc entraîner des modifications dans les protéines PB1, PB1F2 et /ou PB1N40.

Des virus A/PR/8/34 comprenant un gène PB1 modifié sont obtenus par mutagénèse du gène PB1 de A/PR/8/34 et notamment par l'introduction de mutations dans la séquence du gène PB1 ou par le remplacement du gène PB1 de A/PR/8/34 par le gène PB1 d'une autre souche de virus influenza.

Les termes « glycoprotéines HA-NA » réfèrent aux glycoprotéines hémagglutinine (HA) et aux glycoprotéines neuraminidase (NA). Il s'agit des protéines antigéniques spécifiques à la surface des virus influenza de type A pouvant induire la production d'anticorps neutralisants. Les virus influenza de type A se subdivisent en fonction de leur association d'hémagglutinine et de neuraminidase, il existe 16 sous-types HA et 9 sous-types NA.

Grâce à la modification de leur gène PB1, les virus influenza A/PR/8/34 modifiés ainsi que les virus influenza réassortants vaccinaux comportant un fond génétique du virus A/PR/8/34 modifié de la présente invention ont une quantité de glycoprotéines HA-NA accrue à leur surface, par rapport à la quantité de glycoprotéines HA-NA habituellement présente à la surface des virus A/PR/8/34 ne portant pas de gène PB1 modifié.

De préférence, la quantité de glycoprotéines HA-NA à la surface des virus A/PR/8/34 portant un gène PB1 modifié est supérieur à 550, 600 et plus préférentiellement 650 glycoprotéines pour un virion de 100 nm de diamètre. La quantité de glycoprotéines de surface (HA+NA) est déterminée en mesurant la distance entre 4 pieds de glycoprotéines puis est rapportée à la surface d'un virus de 100 nm de diamètre. La mesure de la distance entre 4 pieds de glycoprotéines est réalisée par cryo-microscopie.

Dans un mode de réalisation avantageux, le gène PB1 du virus A/PR/8/34 est remplacé par un gène codant pour la protéine PB1 de la SEQ ID No. 2.

L'invention se rapporte aussi à des virus influenza réassortants ayant le fond génétique d'un virus influenza A/PR/8/34 modifié selon l'invention et comprenant les gènes HA et NA d'un virus influenza ayant les sous-types H3N2, H5N1 ou H5N2. Ces virus réassortants portent donc typiquement 6 gènes du virus A/PR/8/34 modifié (dont le gène PB1 modifié) et deux gènes, les gènes codant pour les glycoprotéines HA et NA, d'une autre souche de virus influenza. Les virus réassortants de la présente invention ont une forte capacité de croissance sur oeufs comme les virus de la souche A/PR/8/34 tout en produisant plus de glycoprotéines HA et NA à leur surface. Ainsi, avec une même quantité de virus réassortant produit, il est possible de produire plus de doses vaccinales. Les gènes codant pour les glycoprotéines HA et NA proviennent de virus de type A et avantageusement de virus influenza sauvages (virus prototype). Avantageusement, les gènes codant pour les glycoprotéines HA et NA proviennent de virus influenza circulants humains ou d'origines animales. Les gènes codant pour les glycoprotéines HA et NA proviennent de virus influenza choisis parmi les virus ayant le sous type H3N2, H5N1 ou H5N2. Dans certains modes de réalisation, l'invention se rapporte à des virus influenza réassortis portant 5 gènes du virus A/PR/8/34 et des gènes PB1, HA et NA provenant d'une ou plusieurs autres souches de virus influenza. De préférence, le gène PB1 du virus A/PR/8/34 est remplacé par le gène PB1 d'une autre souche de virus influenza dont la quantité de glycoprotéines de surface HA-NA est supérieur à 550, 600 ou 650 glycoprotéines pour un virion de 100 nm de diamètre. Selon l'invention, le gène PB1 du virus A/PR/8/34 est remplacé par un gène codant pour la protéine PB1 de la SEQ ID No. 2.

L'invention se rapporte aussi à des procédés mettant en oeuvre des virus A/PR/8/34 modifiés et des virus réassortants ayant le fond génétique du virus A/PR/8/34 modifié pour la production de glycoprotéines HA- NA.

Les virus A/PR/8/34 modifiés selon l'invention et les virus réassortants obtenus avec les virus A/PR/8/34 modifiés sont particulièrement adaptés pour la production de semences vaccinales par amplification sur oeufs de poule fertilisés ou sur cellules ex vivo. Les virus réassortants ayant le fond génétique du virus A/PR/8/34 (6 gènes de A/PR/8/34 dont le gène PB1 modifié) et les gènes HA et NA d'un virus de type sauvage (ou virus prototype) sont particulièrement adaptés pour la production de glycoprotéines HA-NA vaccinales. Les virus réassortants sont amplifiés sur oeuf de poule fertilisé ou sur cellules ex vivo selon des techniques classiques.

Dans des modes de réalisation avantageux, l'invention se rapporte à des procédés pour produire des glycoprotéines HA-NA vaccinales de virus influenza dans lesquels on amplifie sur oeuf ou sur cellule un virus réassortant ayant le fond génétique d'un virus influenza A/PR/8/34 modifié selon l'invention et comprenant les gènes HA et NA codant pour les dites glycoprotéines HA-NA vaccinales. Selon l'invention, les glycoprotéines HA-NA vaccinales sont choisies parmi les glycoprotéines HA-NA de virus influenza ayant les sous-types H3N2, H5N1 ou H5N2 et plus particulièrement parmi les glycoprotéines HA-NA de virus influenza circulants humains ou d'origines animales et ayant les sous-types H3N2, H5N1 ou H5N2. L'invention se rapporte aussi à des procédés pour accroître la quantité de glycoprotéines HA-NA à la surface d'un virus influenza A/PR/8/34 ou d'un virus réassortant A/PR/8/34 comprenant la modification du gène PB1 de ce virus.

Ces modifications du gène PB1 sont décrites ci-dessus.

L'accroissement de la quantité de glycoprotéines HA-NA est mesuré par rapport à la quantité de glycoprotéines présentes à la surface d'un virus A/PR/8/34 ou d'un virus réassortant A/PR/8/34 ne portant pas de gène PB1 modifié.

Dans des modes de réalisation avantageux, cet accroissement permet d'obtenir des virus A/PR/8/34 ou des virus A/PR/8/34 réassortants dont la quantité de glycoprotéines de surface HA-NA est supérieur à 550, 600 ou 650 glycoprotéines pour un virion de 100 nm de diamètre.

De préférence, cette modification comprend la modification du gène PB1 dudit virus influenza A/PR/8/34 ou dudit virus influenza A/PR/8/34 réassortant pour introduire un gène PB1 codant pour une protéine PB1 ayant la séquence SeqIdNo.2. Dans d'autres modes de réalisation, la modification du gène PB1 dudit virus influenza A/PR/8/34 ou dudit virus influenza A/PR/8/34 réassortant comprend le remplacement du gène PB1 du virus influenza A/PR/8/34 par le gène PB1 d'un autre virus influenza dont la quantité de glycoprotéines de surface HA-NA est supérieur à 550, 600 ou 650 glycoprotéines pour un virion de 100 nm de diamètre, à savoir le remplacement du gène PB1 du virus influenza A/PR/8/34 par un gène PB1 codant pour une protéine PB1 ayant la séquence SEQ ID No.2.

L'invention se rapporte aussi à des procédés pour améliorer le rendement de la production de glycoprotéines HA-NA vaccinales de virus influenza par amplification dans des oeufs ou sur cellule ex vivo de virus réassortant vaccinaux ayant un fond génétique A/PR/8/34 dans lesquels on accroît la quantité de glycoprotéines HA-NA à la surface dudit virus réassorti par la modification selon l'invention du gène PB1 de A/PR/8/34.

L'invention se rapporte aussi à un procédé pour préparer un virus réassortant vaccinal pour la production de glycoprotéines HA-NA vaccinales dans lequel on combine 6 gènes d'un virus A/PR/8/34 modifié selon l'invention avec deux gènes codant pour les glycoprotéines vaccinales HA et NA d'un virus influenza prototype (virus de type sauvage).

Un des avantages de l'invention est d'augmenter l'immunogénicité des doses vaccinales par surexpression de NA par rapport à une quantité fixe de HA produite.

Ainsi, l'invention concerne aussi un procédé pour accroître l'immunogénicité d'une dose vaccinale comprenant des glycoprotéines HA-NA de virus influenza en élevant le rapport NA/HA des glycoprotéines produites dans lequel les glycoprotéines sont produites par amplification sur oeufs ou sur cellule ex vivo d'un virus réassortants ayant le fond génétique d'un virus A/PR/8/34 modifié selon l'invention.

### EXEMPLES

### Exemple 1 : Production de virus recombinés par génétique inverse

Nous avons produits 9 virus recombinés par génétique inverse selon le protocole développé dans notre laboratoire. Les compositions génomiques des virus recombinés sont les suivantes (Tableau 1) :
- Virus H3N2 A/Moscow/10/99 (MO)
- Virus H1N1 A/PR/8/34 (PR8)
- Virus A réassortant vaccinal PR8 "classique" comprenant les segment de gene HA et NA de MO sur le fond génétique du virus PR8
- Virus B réassortant vaccinal PR8 comprenant les segment de gene HA, NA et PB1 de MO sur le fond génétique du virus PR8.
- Virus C PR8 comportant le segment de gene PB1 de MO
- Virus D réassortant vaccinal PR8 « classique » comprenant les segments de gene HA et NA du virus aviaire H5N2 A/Finch/England/2051/91 (Virus FI)
- Virus E réassortant vaccinal PR8 comprenant les segment de gene HA, NA de FI et le segment de gene PB1 de MO sur le fond génétique du virus PR8.
- Virus F réassortant vaccinal PR8 « classique » comprenant les segments de gene HA et NA du virus humain H3N2 A/California/10/04 (Virus CAL)
- Virus G réassortant vaccinal PR8 comprenant les segments de gene HA, NA de CAL et le segment de gene PB1 de MO sur le fond génétique du virus PR8.

La production de virus recombiné *in vitro* repose sur la transfection de cellules 293T par 8 plasmides comportant chacun un segment de gène. La production des virus a été réalisée au laboratoire avec ces propres outils moléculaires et cellulaires. Les rendements de production obtenus pour les 9 virus recombinés sont similaires sur cellules et conformes à une production normale de virus recombinés.

**Tableau 1 : Composition génomique des virus recombinés produits par génétique inverse**

| Virus | Composition génomique | | | | | | | |
|---|---|---|---|---|---|---|---|---|
| | HA | NA | M | NS | PB1 | PB2 | PA | NP |
| PR8 | PR8 | PR8 | PR8 | PR8 | PR8 | PR8 | PR8 | PR8 |
| MO | MO | MO | MO | MO | MO | MO | MO | MO |
| A | MO | MO | PR8 | PR8 | PR8 | PR8 | PR8 | PR8 |
| B | MO | MO | PR8 | PR8 | MO | PR8 | PR8 | PR8 |
| C | PR8 | PR8 | PR8 | PR8 | MO | PR8 | PR8 | PR8 |
| D | FI | FI | PR8 | PR8 | PR8 | PR8 | PR8 | PR8 |
| E | FI | FI | PR8 | PR8 | MO | PR8 | PR8 | PR8 |
| F | CAL | CAL | PR8 | PR8 | PR8 | PR8 | PR8 | PR8 |
| G | CAL | CAL | PR8 | PR8 | MO | PR8 | PR8 | PR8 |

| | | | | | | | | |
|---|---|---|---|---|---|---|---|---|
| PR8 : A/PuertoRico/10/34 H1N1 MO : A/Moscou/10/99 H3N2 FI : A/Finch/England/2051/91 CAL : A/California/10/04 H3N2 | | | | | | | | |

### Exemple 2 : Analyse comparative de la densité des glycoprotéines HA-NA à la surface des virus recombinés par cryo-microscopie (Tableau 2)

Les différents virus produits par génétique inverse sont amplifiés et purifiés sur coussin de sucrose. Les virus purifiés déposés sur grille sont congelés a très basse température et observés par microscopie électronique à basse température.

La densité des glycoprotéines de surface (GP) est déterminée par mesure de la distance entre 4 pieds de glycoprotéines (logiciel d'analyse d'image ImageJ) et rapportée à la surface d'un virus de 100 nm de diamètre selon la formule suivante (4piR² avec R=50 nm). Résultats :
Virus PR8 : 500 GPs à la surface d'un virus de 100 nm de diametre.
Virus MO : 689 GPs à la surface d'un virus de 100 nm de diametre.
Virus A : 500 GPs à la surface d'un virus de 100 nm de diametre.
Virus B : 700 GPs à la surface d'un virus de 100 nm de diametre.
Virus C : 598 GPs à la surface d'un virus de 100 nm de diametre.
Virus D : 498 GPs à la surface d'un virus de 100 nm de diametre.
Virus E : 640 GPs à la surface d'un virus de 100 nm de diametre.
Virus F : 502 GPs à la surface d'un virus de 100 nm de diametre.
Virus G : 703 GPs à la surface d'un virus de 100 nm de diametre.

**Tableau 2. Quantité de glycoprotéines (GP) et activité enzymatique NA**

| Virus | Quantité de GP/virus de 100 nm | Activité NA (RFU/10E5 virus) |
|---|---|---|
| PR8 | 500 | 14234 |
| MO | 689 | 29340 |
| A | 500 | 14600 |
| B | 700 | 64000 |
| C | 598 | 63540 |
| D | 498 | 8450 |
| E | 640 | 36542 |
| F | 502 | 13897 |
| G | 703 | 63470 |

- Il y a une différence significative en termes de quantité de glycoprotéines entre le virus recombiné PR8 H1N1 et le virus recombiné MO H3N2 (+ 35% pour le virus H3N2).
- Le virus recombiné A, correspondant à une composition vaccinale classique, présente une quantité de GP similaire au virus PR8.
- Le virus recombiné B (comportant le gene PB1 de MO) présentent une quantité de GP à leur surface identique à celle observée pour le virus MO.

Le gène PB1 permet d'obtenir des virus recombinés vaccinaux comportant une quantité de GP égale à 700 GP/virions de 100 nm de diamètre.
- Le virus recombiné C présentent une quantité de GP à leur surface supérieure à celle observée pour le virus PR8 (20%).
- L'augmentation de la quantité de GP due au segment de gene PB1 MO a été retrouvé pour les virus recombinés F et G présentant à leurs surfaces des GP H3N2 de la souche CAL.
- L'augmentation de la quantité de GP mediée par le segment de gene PB1 de MO a été observée pour des virus réassortant vaccinaux presentant des GP aviaires du virus FI H5N2 (virus D et E).

### Exemple 3 : Détermination et comparaison de l'activité neuraminidase (NA) des virus recombinés (Tableau 2).

La détermination de l'activité NA a été réalisée avec un protocole expérimental développé par le laboratoire (Ferraris et al. Vaccine 2006). L'activité NA étant déterminée pour une quantité de virus donnée (RFUs pour 10^{E}5 virus), les variations observées reflètent la quantité de protéines NA à la surface des virus (pour une NA identique).

Les variations des activités NA RFUs (pour les virus comportant la neuraminidase humaine N2) sont indiquées ci-dessous :
MO : 29340 RFUs pour 10E5 virus
Virus A : 14600 RFUs pour 10E5 virus
Virus B : 58000 RFUs pour 10E5 virus
Virus F : 13897 RFUs pour 10E5 virus
Virus G : 63470 RFUs pour 10E5 virus.

Une augmentation de la quantité de NA est observée pour les virus recombinés B et G par rapport aux virus recombinés A et F qui sont les virus réassortant vaccinaux classiques. Les variations des activités NA RFUs (pour les virus comportant la neuraminidase aviaire N2) sont indiquées ci-dessous :
Virus D : 8450 RFUs pour 10E5 virus
Virus E : 36542 RFUs pour 10E5 virus.

Une augmentation de la quantité de NA est observée pour le virus recombiné E (segment de gene PB1 MO) par rapport au virus recombiné D qui est les virus réassortant vaccinal classique.

Les variations des activités NA RFUs (pour les virus comportant la neuraminidase N1) sont indiquées ci-dessous :
Virus PR8 : 14234 RFUs pour 10E5 virus
Virus C : 63540 RFUs pour 10E5 virus.

Une augmentation de la quantité de NA est observée pour le virus recombiné C (segment de gene PB1 MO) par rapport au virus PR8.

En conclusion, le virus réassortant vaccinal classique comportant le gène PB1 du virus MO présente une activité enzymatique NA supérieure à celle mesurée pour le reassortant vaccinal classique.

L'introduction du gène PB1 du virus MO dans le fond génétique du virus réassortant vaccinal classique permet :
- une augmentation du nombre de GP à la surface des virus (+20 à 35%)
- une augmentation du nombre de NA à la surface des virus (augmentation de l'activité NA).

### Exemple 4 : Détermination des séquences spécifiques de PB1 H3N2 responsables des propriétés présentées ci-dessus, par comparaison de séquences en acides aminés entre les virus H1N1 PR8 et H3N2 MO

L'alignement des séquences protéiques des protéines PB1 montre 30 acides aminés différents entre PR8 et MO.
Une stratégie de mutagénèse dirigée permettant de modifier la séquence protéique de la protéine PB1 PR8 pour obtenir la séquence protéique de la protéine PB1 MO a permis de mettre en évidence une liste de mutations qui fait l'objet de cette demande de brevet.
Liste des mutations : 188 (K→E), 205 (M→I), 212 (L→V), 216 (S→G), 398 (E→D), 486 (R→K), 563 (I→R), 576 (I→L), 581 (E→D), 584 (R→Q), 586 (K→R), 617 (D→N), 621 (Q→R), 682 (V→I) et 691 (R→K).

Uns strategie experimentale plus fine a permis d'identifier deux mutations impliquées dans le processus biologique décrit. L'introduction des mutations I563R et V682I dans la protéine PB1 PR8 (virus A) permet l'obtention du phenotype (quantité de GP et activité NA) similaire à celui observé pour le virus B.

### REFERENCES

Ferraris, O., Kessler, N., Valette, M., Lina, B., 2006. Evolution of the susceptibility to antiviral drugs of A/H3N2 influenza viruses isolated in France from 2002 to 2005. Vaccine 24, 6656-6659
Wanitchang et al., Virus Research, 147, 145-148, 2010

## Revendications

1. Virus influenza A/PR/8/34 dont la quantité de glycoprotéines HA-NA (hémagglutinine et neuraminidase) à la surface est supérieure à 550 glycoprotéines pour un virion de 100 nm de diamètre, comprenant un gène PB1 d'un virus influenza H3N2 codant pour une protéine PB1 ayant la séquence SEQ ID NO : 2 , et les gènes HA et NA dudit virus influenza A/PR/8/34 ou les gènes HA et NA d'un virus influenza ayant les sous-types H3N2, H5N1 ou H5N2.

2. Procédé pour produire des glycoprotéines HA-NA vaccinales de virus influenza **caractérisé en ce que** l'on amplifie sur oeuf ou cellule ex vivo un virus influenza selon la revendication 1 et comprenant les gènes HA et NA codant pour lesdites glycoprotéines HA-NA vaccinales.

3. Procédé pour accroître la quantité de glycoprotéines HA-NA à la surface d'un virus influenza A/PR/8/34 **caractérisé en ce qu'**il comprend la modification du gène PB1 codant pour une protéine PB1 de séquence SEQ ID NO : 1 dudit virus influenza A/PR/8/34 par le gène PB1 d'un virus influenza H3N2 codant pour une protéine PB1 ayant la séquence SEQ ID NO : 2 , et **en ce que** le-dit virus influenza A/PR/8/34 comprend les gènes HA et NA dudit virus influenza A/PR/8/34 ou les gènes HA et NA d'un virus influenza ayant les sous-types H3N2, H5N1 ou H5N2.

4. Procédé pour accroître l'immunogénicité d'une dose vaccinale comprenant des glycoprotéines HA-NA de virus influenza en élevant le rapport NA/HA des glycoprotéines produites dans lequel les glycoprotéines sont produites par amplification sur oeufs ou cellules ex vivo d'un virus influenza selon la revendication 1.

## Patentansprüche

1. Influenza-Virus A/PR/8/34, dessen Menge an Glykoproteinen HA-NA (Hämagglutinin und Neuraminidase) auf der Oberfläche 550 Glykoproteine für ein Virion mit einem Durchmesser von 100 nm übersteigt, umfassend ein Gen PB1 eines Influenza-Virus H3N2, codierend für ein Protein PB1, mit der Sequenz SEQ ID NO: 2, und die Gene HA und NA des Influenza-Virus A/PR/8/34 oder die Gene HA und NA eines Influenza-Virus mit den Untertypen H3N2, H5N1 oder H5N2.

2. Verfahren zur Herstellung der Influenza-Virus-Impf-Glykoproteine HA-NA, **dadurch gekennzeichnet, dass** ein Influenza-Virus nach Anspruch 1, umfassend die Gene HA und NA, codierend für die Impf-Glykoproteine HA-NA, auf Ei oder ex vivo-Zelle amplifiziert wird.

3. Verfahren zur Erhöhung der Menge an Glycoproteinen HA-NA auf der Oberfläche eines Influenza-Virus A/PR/8/34, **dadurch gekennzeichnet, dass** es die Modifizierung des Gens PB1, codierend für ein Protein PB1 der Sequenz SEQ ID NO: 1 des Influenza-Virus A/PR/8/34 durch das Gen PB1 eines Influenza-Virus H3N2, codierend für ein Protein PB1 mit der Sequenz SEQ ID NO: 2, umfasst, und dass das Influenza-Virus A/PR/8/34 die Gene HA und NA des Influenza-Virus A/PR/8/34 oder die Gene HA und NA eines Influenza-Virus mit den Untertypen H3N2, H5N1 oder H5N2 umfasst.

4. Verfahren zur Verstärkung der Immungenizität einer Impfdosis, umfassend Influenza-Virus-Glykoproteine HA-NA, durch Erhöhen des Verhältnisses NA/HA der erzeugten Glykoproteine, wobei die Glykoproteine durch Amplifizieren auf Eiern oder ex vivo-Zellen eines Influenza-Virus nach Anspruch 1 erzeugt werden.

## Claims

1. An influenza A/PR/8/34 virus, whose quantity of HA-NA (hemagglutinin and neuraminidase) glycoproteins on the surface is greater than 550 glycoproteins for a virion of 100 nm in diameter, comprising a PB1 gene of an H3N2 influenza virus coding for a PB1 protein having the sequence SEQ ID NO: 2, and the HA and NA genes of said influenza A/PR/8/34 virus or the HA and NA genes of an influenza virus having the H3N2, H5N1 or H5N2 subtypes.

2. A method for producing HA-NA vaccine glycoproteins of influenza virus, **characterized in that** an influenza virus is ex-vivo amplified on eggs or cells according to claim 1, and comprising the HA and NA genes coding for said HA-NA vaccine glycoproteins.

3. A method for increasing the quantity of HA-NA glycoproteins on the surface of an influenza A/PR/8/34 virus, **characterized in that** it comprises the modification of the PB1 gene coding for a PB1 protein of sequence SEQ ID NO: 1 of said influenza A/PR/8/34 virus by the PB1 gene of an H3N2 influenza virus coding for a PB1 protein having the sequence SEQ ID NO: 2, and **in that** said influenza A/PR/8/34 virus comprises the HA and NA genes of said influenza A/PR/8/34 virus or the HA and NA genes of an influenza virus having the H3N2, H5N1 or H5N2 subtypes.

4. A method for increasing the immunogenicity of a dose of vaccine comprising HA-NA glycoproteins of influenza virus by raising the NA/HA ratio of the glycoproteins produced, wherein the glycoproteins are produced by ex-vivo amplification on eggs or cells, of an influenza virus according to claim 1.
